# EUROPEAN PATENT APPLICATION

(11) **EP 0 849 276 A1**
(43) Date of publication of application: **24.06.1998**
(21) Application number: 97310190.0
(22) Date of filing: 16.12.1997
(51) Int. Cl.: C07K 14/575, A61K 38/22

(54) **Anti-obesity proteins**

(30) Priority: 20.12.1996 US 33561 P
(71) Applicant: ELI LILLY AND COMPANY, Indianapolis, Indiana 46285 (US)
(72) Inventor: Beals, John Michael, Indianapolis, Indiana 46278 (US); Chance, Ronald Eugene, Westfield, Indiana 46074 (US); Hoffmann, James Arthur, Greenwood, Indiana 46143 (US); Millican, Rohn Lee, Jr., Indianapolis, Indiana 46254 (US)
(74) Representative: Denholm, Anna Marie

(57) **Abstract**

The present invention provides anti-obesity proteins wherein at least one of the Trp 100 or Trp 138 is replaced by Glu, Asp, His, Lys or Arg, which when administered to a patient regulate fat tissue. Accordingly, such agents allow patients to overcome their obesity handicap and live normal lives with much reduced risk for type 2 diabetes, cardiovascular disease, and cancer.

## Description

### Field of the Invention

The present invention is in the field of human medicine, particularly in the treatment of obesity and disorders associated with obesity. Most specifically the invention relates to anti-obesity proteins that when administered to a patient regulate fat tissue.

### Background of the Invention

Obesity, and especially upper body obesity, is a common and very serious public health problem in the United States and throughout the world. According to recent statistics, more than 25% of the United States population and 27% of the Canadian population are overweight [Kuczmarski, R. J., *Amer. J. of Clin. Nutr.* 55:495S-502S (1992); Reeder, B. A., *et. al., Can. Med. Assoc. J.,* 146:2009-2019 (1992)]. Upper body obesity is the strongest risk factor known for people with type 2 diabetes, and is a strong risk factor for cardiovascular disease and cancer as well. Recent estimates for the medical cost of obesity are $150,000,000,000 world wide. The problem has become serious enough that the surgeon general has begun an initiative to combat the ever increasing adiposity rampant in American society.

Much of this obesity-induced pathology can be attributed to the strong association with dyslipidemia, hypertension, and insulin resistance. Many studies have demonstrated that reduction in obesity by diet and exercise reduces these risk factors dramatically. Unfortunately, these treatments are largely unsuccessful with a failure rate reaching 95%. This failure may be due to the fact that the condition is strongly associated with genetically inherited factors that contribute to increased appetite, preference for highly caloric foods, reduced physical activity, and increased lipogenic metabolism. This indicates that people inheriting these genetic traits are prone to becoming obese regardless of their efforts to combat the condition. Therefore, a pharmacological agent that can correct this adiposity handicap and allow the physician to successfully treat obese patients in spite of their genetic inheritance is needed.

Physiologists have postulated for years that, when a mammal overeats, the resulting excess fat signals to the brain that the body is obese which, in turn, causes the body to eat less and burn more fuel [Hervey, G. R., *Nature* 222:629-631 (1969)]. This "feedback" model is supported by parabiotic experiments, which implicate a circulating hormone controlling adiposity.

The *ob/ob* mouse is a model of obesity and diabetes that is known to carry an autosomal recessive trait linked to a mutation in the sixth chromosome. Recently, Zhang, Y. and co-workers published the positional cloning of the mouse gene linked with this condition [Zhang, Y., *et al. Nature* 372:425-432 (1994)]. This report disclosed a gene coding for a 167 amino acid protein with a 21 amino acid signal peptide that is exclusively expressed in adipose tissue. The rat obese gene was subsequently cloned and expressed [Murakami, T. *et al., Biochem. Biophys. Res. Comm.* 209:944-952 (1995)]. The protein, which is apparently encoded by the *ob* gene, is now speculated to be the adiposity regulating hormone.

The protein encoded by the *ob* gene is pharmacologically active, but the physical properties of the protein are less than desirable. The human protein, for example, is prone to precipitation and aggregation in both a pharmaceutical formulation and under physiological conditions. Formulations of a protein containing a precipitate, or that permit protein precipitation after injection, increase the risk of producing an immunological response in the patient and may result in irritation at the site of injection. Accordingly, there remains a need to develop pharmacological agents that demonstrate pharmacological activity and provide improved physical and chemical stability.

Applicants have discovered that the aggregation observed in the native human protein is due, in part, to hydrophobic interactions at the surface of the protein, particularly at residues 100 and 138. Applicants have further discovered that by substituting these positions with charged amino acids, the propensity of the ob protein to aggregate is dramatically reduced, which provides a much improved pharmacological agent. Accordingly, the present invention provides biologically active obesity proteins. Such agents allow patients to overcome their obesity handicap and live normal lives with a more normalized risk for type 2 diabetes, cardiovascular disease, and cancer.

### Summary of the Invention

The present invention is directed to a protein of the Formula (I): 
(SEQ ID NO:1)
5 10 15
Val Pro Ile Gln Lys Val Gln Asp Asp Thr Lys Thr Leu Ile Lys Thr

20 25 30
Ile Val Thr Arg Ile Xaa Asp Ile Ser His Thr Xaa Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Xaa Xaa Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro
 
145
Gly Cys (I)
 wherein:
Xaa at position 22 is Asn or Ser;
Xaa at position 28 is Gln or absent;
Xaa at position 72 in Asn, Gln, Glu, or Asp;
Xaa at position 73 is Val or Met;
said protein having at least one of the following substitutions:
Trp at position 100 is replaced with Glu, Asp, His, Lys, or Arg;
Trp at position 138 is replaced with Glu, Asp, His, Lys, or Arg;
or a pharmaceutically acceptable salt thereof.

The invention further provides a method of treating obesity or those conditions associated with obesity, which comprises administering to a mammal in need thereof a protein of the Formula (I).

The invention further provides a pharmaceutical formulation, which comprises a protein of the Formula (I) together with one or more pharmaceutically acceptable diluents, carriers, or excipients therefor.

The invention further provides proteins of the Formula (I) having additionally a leader sequence bonded to the N-terminus of the protein of Formula I. Such proteins are useful for their anti-obesity activity and as intermediates in the preparation of proteins of the Formula (I).

The invention further provides DNA encoding a protein of Formula (I), or a protein of Formula (I) having a leader sequence.

An additional embodiment of the present invention is a process for producing a protein of Formula (I), which comprises:
(a) transforming a host cell with DNA that encodes the protein of Formula (I) or a protein of Formula (I) having a leader sequence;
(b) culturing the host cell under conditions permitting expression of the protein;
(c) recovering the expressed protein; and, optionally,
(d) cleaving enzymatically the leader sequence to produce the protein of Formula (I).

The invention further provides a protein for use in treating obesity or conditions associated with obesity, and also a protein for use in manufacturing a medicament for treating obesity or conditions associated with obesity.

### Detailed Description

For purposes of the present invention, as disclosed and claimed herein, the following terms and abbreviations are defined as follows:

Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively, when they occur in RNA molecules. In double stranded DNA, base pair may refer to a partnership of A with T or C with G. In a DNA/RNA heteroduplex, base pair may refer to a partnership of T or U with A or C with G.

FMOC - an abbreviation for 9-. fluorenymethoxycarbony.

Immunoreactive Protein(s) -- a term used to collectively describe antibodies, fragments of antibodies capable of binding antigens of a similar nature as the parent antibody molecule from which they are derived, and single chain polypeptide binding molecules [Bird, E. R., et al., PCT Application No. PCT/US 87/02208, International Publication No. WO 88/01649, published March 10, 1988].

Plasmid -- an extrachromosomal self-replicating genetic element.

PAM - an abbreviation for 4-hydroxymethyl-phenylacetamidomethyl.

PMSF -- an abbreviation for phenylmethylsulfonyl fluoride.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e., the correct "reading frame" must be maintained.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- any recombinant DNA cloning vector in which a promoter has been incorporated.

Replicon -- A DNA sequence that controls and allows for autonomous replication of a plasmid or other vector.

TFA - an abbreviation for trifluoroacetic acid.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Tris -- an abbreviation for tris(hydroxymethyl)-aminomethane.

Treating -- describes the management and care of a patient for the purpose of combating the disease, condition, or disorder and includes the administration of a compound of present invention to prevent the onset of the symptoms or complications, alleviating the symptoms or complications, or eliminating the disease, condition, or disorder. Treating obesity, therefore, includes the inhibition of food intake, the inhibition of weight gain, and inducing weight loss in patients in need thereof.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

X-gal -- an abbreviation for 5-bromo-4-chloro-3-indolyl beta-D-galactoside.

The amino acid abbreviations are accepted by the United States Patent and Trademark Office as set forth in 37 C.F.R. § 1.822 (b) (2) (1993). Unless otherwise indicated the amino acids are in the L configuration.

As noted above the present invention provides a protein of the Formula (I): 
                    5                   .  10                      15
Val Pro Ile Gln Lys Val Gln Asp Asp Thr Lys Thr Leu Ile Lys Thr

                20                  25                  30
Ile Val Thr Arg Ile Xaa Asp Ile Ser His Thr Xaa Ser Val Ser Ser

            35                  40                  45
Lys Gln Lys Val Thr Gly Leu Asp Phe Ile Pro Gly Leu His Pro Ile
 
    50                  55                  60
Leu Thr Leu Ser Lys Met Asp Gln Thr Leu Ala Val Tyr Gln Gln Ile

65                  70                  75                  80
Leu Thr Ser Met Pro Ser Arg Xaa Xaa Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys    (SEQ ID NO:1)                                        (I)
 wherein:
Xaa at position 22 is Asn or Ser;
Xaa at position 28 is Gln or absent;
Xaa at position 72 in Asn, Gln, Glu, or Asp;
Xaa at position 73 is Val or Met;
said protein having at least one of the following substitutions: Trp at position 100 is replaced with Glu, Asp, His, Lys, or Arg; or Trp at position 138 is replaced with Glu, Asp, His, Lys, or Arg; or a pharmaceutically acceptable salt thereof.

Preferred proteins of the present invention are those of Formula I, wherein:
Xaa at position 22 is Asn;
Xaa at position 28 is Gln;
Xaa at position 72 in Asn or Asp; and
Xaa at position 73 is Val.

Other preferred proteins are those wherein Trp at position 100 is replaced with Glu or Asp; or Trp at position 138 is replaced with Glu or Asp. Particularly preferred are proteins of Formula I wherein Xaa at position 72 is Asp. Additional preferred proteins are those wherein Trp at position 100 is replaced with His, Lys, or Arg. Other preferred proteins of the Formula I are those wherein Trp at position 100 is replaced with Lys or Arg; or Trp at position 138 is replaced with Lys or Arg.

The most preferred species of the present invention include the species of SEO ID NO:2-12:

5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Asp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys  (SEQ ID NO:2)

                    5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Glu Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

130                 135                 140
Leu Gln Gly Ser Leu Gln Asp Met Leu Trp Gln Leu Asp Leu Ser Pro

145
Gly Cys  (SEQ ID NO:3)

                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Glu Gln Leu Asp Leu Ser Pro

145
Gly Cys  (SEQ ID NO:4)


                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Glu Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

130                 135                 140
Leu Gln Gly Ser Leu Gln Asp Met Leu Asp Gln Leu Asp Leu Ser Pro

145
Gly Cys  (SEQ ID NO:5)

                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asp Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Asp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys  (SEQ ID NO:6)

                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
Ser Leu Pro Lys Thr Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

130                 135                 140
Leu Gln Gly Ser Leu Gln Asp Met Leu Trp Gln Leu Asp Leu Ser Pro

145
Gly Cys  (SEQ ID NO:7)

                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
Ser Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Lys Gln Leu Asp Leu Ser Pro

145
Gly Cys  (SEQ ID NO:8)


                5                   10                  15
Met Arg Val Pro Ile Gln Lys Val Gln Asp Asp Thr Lys Thr Leu Ile

            20                  25                  30
Lys Thr Ile Val Thr Arg Ile Asn Asp Ile Ser His Thr Gln Ser Val

        35                  40                  45
Ser Ser Lys Gln Lys Val Thr Gly Leu Asp Phe Ile Pro Gly Leu His

    50                  55                  60
Pro Ile Leu Thr Leu Ser Lys Met Asp Gln Thr Leu Ala Val Tyr Gln

65                  70                  75                  80
Gln Ile Leu Thr Ser Met Pro Ser Arg Asp Val Ile Gln Ile Ser Asn

                85                  90                  95
Asp Leu Glu Asn Leu Arg Asp Leu Leu His Val Leu Ala Phe Ser Lys

            100                 105                 110
Ser Cys His Leu Pro Glu Ala Ser Gly Leu Glu Thr Leu Asp Ser Leu

        115                 120                 125
Gly Gly Val Leu Glu Ala Ser Gly Tyr Ser Thr Glu Val Val Ala Leu

130                 135                 140
Ser Arg Leu Gln Gly Ser Leu Gln Asp Met Leu Trp Gln Leu Asp Leu

145
Ser Pro Gly Cys  (SEQ ID NO:9)

                5                   10                  15
Met Arg Val Pro Ile Gln Lys Val Gln Asp Asp Thr Lys Thr Leu Ile

            20                  25                  30
Lys Thr Ile Val Thr Arg Ile Asn Asp Ile Ser His Thr Gln Ser Val

        35                  40                  45
Ser Ser Lys Gln Lys Val Thr Gly Leu Asp Phe Ile Pro Gly Leu His

    50                  55                  60
Pro Ile Leu Thr Leu Ser Lys Met Asp Gln Thr Leu Ala Val Tyr Gln

65                  70                  75                  80
Gln Ile Leu Thr Ser Met Pro Ser Arg Asp Val Ile Gln Ile Ser Asn

                85                  90                  95
Asp Leu Glu Asn Leu Arg Asp Leu Leu His Val Leu Ala Phe Ser Lys

            100                 105                 110
Ser Cys His Leu Pro Asp Ala Ser Gly Leu Glu Thr Leu Asp Ser Leu

        115                 120                 125
Gly Gly Val Leu Glu Ala Ser Gly Tyr Ser Thr Glu Val Val Ala Leu

    130                 135                 140
Ser Arg Leu Gln Gly Ser Leu Gln Asp Met Leu Trp Gln Leu Asp Leu

145
Ser Pro Gly Cys  (SEQ ID NO:10)

                5                   10                  15
Met Arg Val Pro Ile Gln Lys Val Gln Asp Asp Thr Lys Thr Leu Ile

            20                  25                  30
Lys Thr Ile Val Thr Arg Ile Asn Asp Ile Ser His Thr Gln Ser Val

        35                  40                  45
Ser Ser Lys Gln Lys Val Thr Gly Leu Asp Phe Ile Pro Gly Leu His

    50                  55                  60
Pro Ile Leu Thr Leu Ser Lys Met Asp Gln Thr Leu Ala Val Tyr Gln

65                  70                  75                  80
Gln Ile Leu Thr Ser Met Pro Ser Arg Asn Val Ile Gln Ile Ser Asn

                85                  90                  95
Asp Leu Glu Asn Leu Arg Asp Leu Leu His Val Leu Ala Phe Ser Lys

            100                 105                 110
Ser Cys His Leu Pro Glu Ala Ser Gly Leu Glu Thr Leu Asp Ser Leu

        115                 120                 125
Gly Gly Val Leu Glu Ala Ser Gly Tyr Ser Thr Glu Val Val Ala Leu

130                 135                 140
Ser Arg Leu Gln Gly Ser Leu Gln Asp Met Leu Trp Gln Leu Asp Leu

145

Ser Pro Gly Cys  (SEQ ID NO:11)
                5                   10                  15
Met Arg Val Pro Ile Gln Lys Val Gln Asp Asp Thr Lys Thr Leu Ile

            20                  25                  30
Lys Thr Ile Val Thr Arg Ile Asn Asp Ile Ser His Thr Gln Ser Val

        35                  40                  45
Ser Ser Lys Gln Lys Val Thr Gly Leu Asp Phe Ile Pro Gly Leu His

    50                  55                  60
Pro Ile Leu Thr Leu Ser Lys Met Asp Gln Thr Leu Ala Val Tyr Gln

65                  70                  75                  80
Gln Ile Leu Thr Ser Met Pro Ser Arg Asn Val Ile Gln Ile Ser Asn

                85                  90                  95
Asp Leu Glu Asn Leu Arg Asp Leu Leu His Val Leu Ala Phe Ser Lys

            100                 105                 110
Ser Cys His Leu Pro Asp Ala Ser Gly Leu Glu Thr Leu Asp Ser Leu

        115                 120                 125
Gly Gly Val Leu Glu Ala Ser Gly Tyr Ser Thr Glu Val Val Ala Leu

    130                 135                 140
Ser Arg Leu Gln Gly Ser Leu Gln Asp Met Leu Trp Gln Leu Asp Leu

145

Ser Pro Gly Cys  (SEQ ID NO:12)

The present invention provides biologically active proteins that provide effective treatment for obesity. Applicants have discovered that specific substitutions to the hydrophobic residues on the surface of the protein result in improved properties. These residues could not be predicted from the primary sequence. The proteins having these substitutions are pharmacologically active and have a reduced propensity to aggregate when compared to both the mouse and human forms of the protein. The present invention permits obesity protein to be more readily formulated at higher concentrations, and increases their pharmaceutical elegance, because they are compatible with commonly-used preservatives.

The claimed proteins ordinarily are prepared by recombinant techniques. Techniques for making substitutional mutations at predetermined sites in DNA having a known sequence are well known, for example M13 primer mutagenesis. The mutations that might be made in the DNA encoding the present anti-obesity proteins must not place the sequence out of reading frame and preferably will not create complementary regions that could produce secondary mRNA structure [DeBoer, H.A., *et al.,* European Patent Publication No. 75,444 A2, published March 30, 1983].

The compounds of the present invention may be produced either by recombinant DNA technology or well known chemical procedures, such as solution or solid-phase peptide synthesis, or semi-synthesis in solution beginning with protein fragments coupled through conventional solution methods.

### A. Solid Phase

The synthesis of the claimed proteins may proceed by solid phase peptide synthesis or by recombinant methods. The principles of solid phase chemical synthesis of polypeptides are well known in the art and may be found in general texts in the area such as Dugas, H. and Penney, C., *Bioorganic Chemistry* Springer-Verlag, New York, (1981) 54-59. For example, peptides may be synthesized by solid-phase methodology utilizing an PE-Applied Biosystems 433A peptide synthesizer (Perkin Elmer-Applied Biosystems Division, Foster City, California) and synthesis cycles supplied by Applied Biosystems. Boc amino acids and other reagents are commercially available from PE-Applied Biosystems and other chemical supply houses. Sequential Boc chemistry using double couple protocols are applied to the starting p-methyl benzhydryl amine resins for the production of C-terminal carboxamides. For the production of C-terminal acids, the corresponding PAM resin is used. Arginine, Asparagine, Glutamine, Histidine, and Methionine are coupled using preformed hydroxy benzotriazole esters. The following side chain protection may be used:
Arg, Tosyl
Asp, cyclohexyl or benzyl
Cys, 4-methylbenzyl
Glu, cyclohexyl
His, benzyloxymethyl
Lys, 2-chlorobenzyloxycarbonyl
Met, sulfoxide
Ser, Benzyl
Thr, Benzyl
Trp, formyl
Tyr, 4-bromo carbobenzoxy
Boc deprotection may be accomplished with trifluoroacetic acid (TFA) in methylene chloride. Formyl removal from Trp is accomplished by treatment of the peptidyl resin with 20% piperidine in dimethylformamide for 60 minutes at 4°C. Met(O) can be reduced by treatment of the peptidyl resin with TFA/dimethylsulfide/conc. HCl (95/5/1) at 25°C for 60 minutes. Following the above pre-treatments, the peptides may be further deprotected and cleaved from the resin with anhydrous hydrogen fluoride containing a mixture of 10% m-cresol or m-cresol/10% p-thiocresol or m-cresol/p-thiocresol/dimethyl-sulfide. Cleavage of the side chain protecting group(s) and of the peptide from the resin is carried out at 0°C or below, preferably -20°C for thirty minutes followed by thirty minutes at 0°C. After removal of the HF, the peptide/resin is washed with ether. The peptide is extracted with glacial acetic acid and lyophilized. Purification is accomplished by reverse-phase C18 chromatography in 0.1% TFA with a gradient of increasing acetonitrile concentration, e.g., a 2.2 cm X 25 cm Vydac™ column (The Separations Group, Inc., Hesperia, CA).

One skilled in the art recognizes that the solid phase synthesis could also be accomplished using the FMOC strategy and a TFA/scavenger cleavage mixture.

### B. Recombinant Synthesis

The claimed proteins may also be produced by recombinant methods. Recombinant methods are preferred if a high yield is desired. The basic steps in the recombinant production of protein include:
a) construction of a synthetic or semi-synthetic (or isolation from natural sources) DNA encoding the claimed protein,
b) integrating the coding sequence into an expression vector in a manner suitable for the expression of the protein either alone or as a fusion protein,
c) transforming an appropriate eukaryotic or prokaryotic host cell with the expression vector, and
d) recovering and purifying the recombinantly produced protein.

### a. Gene Construction

Synthetic genes, the *in vitro* or *in vivo* transcription and translation of which will result in the production of the protein may be constructed by techniques well known in the art. Owing to the natural degeneracy of the genetic code, the skilled artisan will recognize that a sizable yet definite number of DNA sequences may be constructed which encode the claimed proteins. In the preferred practice of the invention, synthesis is achieved by recombinant DNA technology.

Methodology of synthetic gene construction is well known in the art [Brown, E. L., *et al. Methods in Enzymology,* Academic Press, New York, NY, 68:109-151 (1979)]. The DNA sequence corresponding to the synthetic claimed protein gene may be generated using conventional DNA synthesizing apparatus such as the Applied Biosystems Model 380A or 380B DNA synthesizers (Perkin Elmer, Applied Biosystems Division, Foster City, California).

It may desirable in some applications to modify the coding sequence of the claimed protein so as to incorporate a convenient protease sensitive cleavage site, e.g., between the signal peptide and the structural protein facilitating the controlled excision of the signal peptide from the fusion protein construct.

The gene encoding the claimed protein may also be created by using polymerase chain reaction (PCR). The template can be a cDNA library (commercially available from CLONETECH or STRATAGENE) or mRNA isolated from human adipose tissue. Such methodologies are well known in the art. See, *e.g.,* Maniatis, T., *et al. Molecular Cloning: A Laboratory Manual,* 2^{nd} Ed., Cold Spring Harbor Press, Cold Spring Harbor Laboratory, Cold Spring Harbor, New York (1989).

### b. Direct Expression or Fusion Protein

The claimed protein may be made either by direct expression or as fusion protein comprising the claimed protein followed by enzymatic or chemical cleavage. A variety of peptidases (e.g. trypsin) which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See, e.g.,* Carter, P., Chapter 13 in *Protein Purification: From Molecular Mechanisms to Large-Scale Processes,* Ladisch, M., *et al.* (Eds.) American Chemical Soc., Washington, D.C. (1990).

### c. Vector Construction

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. A synthetic coding sequence is designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. Restriction sites are chosen so as to properly orient the coding sequence with control sequences to achieve proper in-frame reading and expression of the claimed protein.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, *E. coli* is typically transformed using pBR322, a plasmid derived from an *E. coli* species [Bolivar, F., *et al.*, Gene 2:95-113 (1977)]. Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje, R. M., *et al.,* U.S. patent No. 5,304,473, issued, April 19, 1994, the teachings of which are herein incorporated by reference. The gene encoding A-C-B proinsulin described in U.S. Patent No. 5,304,473 can be removed from the plasmid pRB182 with restriction enzymes *NdeI* and *BamHI.* The genes encoding the protein of the present invention can be inserted into the plasmid backbone on a *NdeI/BamHI* restriction fragment cassette.

### d. Prokaryotic Expression

In general, prokaryotes are used for cloning of DNA sequences in constructing the vectors useful in the invention. For example, *E. coli* K12 strain 294 (ATCC No. 31446) is particularly useful. Other microbial strains which may be used include *E. coli* B and *E. coli* X1776 (ATCC No. 31537). These examples are illustrative rather than limiting.

Prokaryotes also are used for expression. The aforementioned strains, as well as *E. coli* W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the β-lactamase (vector pGX2907 [ATCC 39344] contains the replicon and β-lactamase gene) and lactose promoter systems [Chang, A. C. Y., *et al., Nature,* 275:617-624 (1978); and Goeddel, D. V., *et al., Nature* 281:544-548 (1979)], alkaline phosphatase, the tryptophan *(trp)* promoter system (vector pATH1 [ATCC 37695] is designed to facilitate expression of an open reading frame as a *trp*E fusion protein under control of the *trp* promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

### e. Eukaryotic Expression

The protein may be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. β-actin promoter. The early and late promoters of the SV40 virus are conveniently obtained as an SV40 restriction fragment which also contains the SV40 viral origin of replication [Fiers, W., *et al., Nature,* 273:113-120 (1978)]. The entire SV40 genome may be obtained from plasmid pBRSV, ATCC 45019. The immediate early promoter of the human cytomegalovirus may be obtained from plasmid pCMBβ (ATCC 77177). Of course, promoters from the host cell or related species also are useful herein.

Transcription of a DNA encoding the claimed protein by higher eukaryotes is increased by inserting an enhancer sequence into the vector. Enhancers are cis-acting elements of DNA, usually about 10-300 bp, that act on a promoter to increase its transcription. Enhancers are relatively orientation and position independent having been found 5' [Laimins, L. A., *et al., Proc. Nat'1 Acad. Sci. (USA)* 78:464-468 (1981)] and 3' [Lusky, M., *et al., Mol. Cell. Bio.* 3:1108-1122 (1983)] to the transcription unit, within an intron [Banerji, J., *et al., Cell* 33:729-740 (1983)] as well as within the coding sequence itself [Osborne, T. F., *et al., Mol. Cell. Bio.* 4:1293-1305 (1984)]. Many enhancer sequences are now known from mammalian genes (globin, RSV, SV40, EMC, elastase, albumin, α-fetoprotein, and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human, or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the *BglII/HindIII* restriction fragment of pJOD-10 [ATCC 68815]), thymidine kinase (herpes simplex virus thymidine kinase is contained on the *BamHI* fragment of vP-5 clone (ATCC 2028) or neomycin (G418) resistance genes, which are obtainable from pNN414 yeast artificial chromosome vector (ATCC 37682). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells [L-M(TK-) ATCC CCL-2.3]. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, [Southern P J., *et al., J. Molec. Appl. Genet.* 1:327-341 (1982)], mycophenolic acid [Mulligan, R. C. *et al., Science* 209:1422-1427 (1980)], or hygromycin [Sugden, B. *et al., Mol Cell. Biol.* 5:410-413 (1985)]. The three examples given above employ bacterial genes under eukaryotic control to convey resistance to the appropriate drug, such as, G418, neomycin (geneticin), xgpt (mycophenolic acid), or hygromycin, respectively.

A preferred vector for eukaryotic expression is pRc/CMV. pRc/CMV is commercially available from Invitrogen Corporation, San Diego, CA. To confirm correct sequences in constructed plasmids, the ligation mixtures are used to transform *E. coli* K12 strain DH5a (ATCC 31446) and successful transformants are selected by antibiotic resistance where appropriate. Plasmids from the transformants are prepared, analyzed by restriction and/or sequenced by the method of Messing, J., *et al., Nucleic Acids Res.* 9:309-321 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, T., *et al. Molecular Cloning: A Laboratory Manual,* 2^{nd} Ed., Cold Spring Harbor Press, Cold Spring Harbor Laboratory, Cold Spring Harbor, New York (1989), or *Current Protocols in Molecular Biology* (1989) and supplements.

Preferred suitable host cells for expressing the vectors encoding the claimed proteins in higher eukaryotes include: African green monkey kidney cell line transformed by SV40 (COS-7, ATCC CRL-1651); transformed human primary embryonal kidney cell line 293 [Graham, F. L. *et al., J. Gen Virol.* 36:59-72 (1977); Harrison, T., *et al., Virology* 77:319-329 (1977); Graham, F. L. *et al., Virology* 86:10-21 (1978)]; baby hamster kidney cells [BHK-21(C-13), ATCC CCL-10; MacPherson, I., *et al, Virology* 16:147-151 (1962)]; Chinese hamster ovary cells [CHO-DHFR⁻ (ATCC CRL-9096)]; mouse Sertoli cells [TM4, ATCC CRL-1715; Mather, J. P., *Biol. Reprod.* 23:243-252 (1980)]; African green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

### f. Yeast Expression

In addition to prokaryotic and mammalian host cells, eukaryotic microorganisms such as yeast may also be used as host cells. *Saccharomyces cerevisiae,* common baker's yeast, is the most commonly-used eukaryotic microorganism for expressing heterologous proteins, although a number of other strains are commonly available. For expression in *Saccharomyces,* the plasmid YRp7, for example, is commonly used [ATCC-40053, Stinchcomb, D. T., et al., *Nature* 282:39-43 (1979); Kingsman, A. J., et al., *Gene* 7:141-152 (1979); Tschumper, G., *et al., Gene* 10:157-166 (1980)]. This plasmid already contains the trp gene which provides a selection marker for a mutant strain of yeast lacking the ability to grow in tryptophan [e.g., ATCC 44076 or PEP4-1; Jones, E. W., *Genetics* 85:23-33 (1977)].

Suitable promoting sequences for use with yeast hosts include the promoters for 3-phosphoglycerate kinase, which is found on plasmid pAP12BD ATCC 53231 [Patel, A. C., *et al.*, U.S. Patent No. 4,935,350, issued June 19, 1990] or other glycolytic enzymes such as enolase, which is found on plasmid pAC1 (ATCC 39532), glyceraldehyde-3-phosphate dehydrogenase, which is derived from plasmid pHcGAPC1 (ATCC 57090, 57091), zymomonas mobilis [Ingram, L.O., et al., U.S. Patent No. 5,000,000 issued March 19, 1991], hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, which is contained on plasmid vector pCL28XhoLHBPV [ATCC 39475; Reddy, V. B., *et al.,* U.S. Patent No. 4,840,896, issued June 20, 1989], glyceraldehyde 3-phosphate dehydrogenase, and enzymes responsible for maltose and galactose utilization, such as, the GAL1 promoter, which may be found on plasmid pRyl21 (ATCC 37658). Suitable vectors and promoters for use in yeast expression are further described in Hitzeman, R. A., *et al.,* European Patent Publication No. 73,657A1, published March 9, 1983. Yeast enhancers such as the UAS Gal from *Saccharomyces cerevisiae,* which is found in conjunction with the CYC1 promoter on plasmid YEpsec--hIlbeta (ATCC 67024), also are advantageously used with yeast promoters.

The following examples are presented to further illustrate the preparation of the claimed proteins. The scope of the present invention is not to be construed as merely consisting of the following examples.

### Example 1

### Vector Construction

A gene of SEQ ID NO:13 is assembled from a ∼220 base pair and a ∼240 base pair segment which are derived from chemically synthesized oligonucleotides. 
(SEQ ID NO:13)
1 CATATGAGGG TACCTATCCA AAAAGTACAA GATGACACCA AAACACTGAT
51 AAAGACAATA GTCACAAGGA TAAATGATAT CTCACACACA CAGTCAGTCT
101 CATCTAAACA GAAAGTCACA GGCTTGGACT TCATACCTGG GCTGCACCCC
151 ATACTGACAT TGTCTAAAAT GGACCAGACA CTGGCAGTCT ATCAACAGAT
201 CTTAACAAGT ATGCCTTCTA GAAACGTGAT ACAAATATCT AACGACCTGG
251 AGAACCTGCG GGATCTGCTG CACGTGCTGG CCTTCTCTAA AAGTTGCCAC
301 TTGCCATGGG CCAGTGGCCT GGAGACATTG GACAGTCTGG GGGGAGTCCT
351 GGAAGCCTCA GGCTATTCTA CAGAGGTGGT GGCCCTGAGC AGGCTGCAGG
401 GGTCTCTGCA AGACATGCTG TGGCAGCTGG ACCTGAGCCC CGGGTGCTAA
451 TAGGATCC
 The 220 base pair segment extends from the *NdeI* site to the *XbaI* site at position 220 within the coding region and is assembled from 7 overlapping oligonucleotides which range in length from between 34 and 83 bases. The 240 base pair segment which extends from the *XbaI* to the *BamHI* site is also assembled from 7 overlapping oligonucleotides which range in length from between 57 and 92 bases.

To assemble these fragments, the respective 7 oligonucleotides are mixed in equimolar amounts, usually at concentrations of about 1-2 picomoles per microliter. Prior to assembly, all but the oligonucleotides at the 5'-ends of the segment are phosphorylated in standard kinase buffer with T4 DNA kinase using the conditions specified by the supplier of the reagents. The mixtures are heated to 95°C and allowed to cool slowly to room temperature over a period of 1-2 hours to ensure proper annealing of the oligonucleotides. The oligonucleotides are then ligated to each other and into an appropriated cloning vector such as pUC18 or pUC 19 using T4 DNA ligase. The buffers and conditions are those recommended by the supplier of the enzyme. The vector for the 220 base pair fragment is digested with *NdeI* and *XbaI,* whereas the vector for the 240 base pair fragment is digested with *XbaI* and *BamHI* prior to use. The ligation mixes are used to transform *E. coli* DH10B cells (commercially available from Life Technologies, producer of GIBCO/BRL products, Grand Island, NY) and the transformed cells are plated on tryptone-yeast plates (TY, Difco, Detroit, MI) containing 100 µg/ml of ampicillin, X-gal and IPTG. Colonies which grow up overnight are grown in liquid TY medium with 100 µg/ml of ampicillin and are used for plasmid isolation and DNA sequence analysis. Plasmids with the correct sequence are kept for the assembly of the complete gene. This is accomplished by gel-purification of the 220 base-pair and the 240 base-pair fragments and ligation of these two fragments into an expression vector such as pRB182 from which the coding sequence for A-C-B proinsulin is deleted and is digested with *NdeI* and *BamHI* prior to use.

Alternatively, plasmid pET30 (Novagen, Madison, WI) can be digested with *NdeI* and *BamHI,* and the desired DNA sequence encoding the proteins of the present invention can be inserted by procedures recognized in the art and described herein. The source of the DNA is synthetic oligonucleotides that are assembled by art recognized methodology and described herein.

### Example 2

Plasmid pOJ722 (NRRL, No. B-21,654) containing the DNA sequence encoding the desired protein, was prepared in a manner analogous to Example 1. The plasmid was digested with *PmlI* and *Bsu36*I*.* The recognition sequences for these enzymes lie within the coding region for the protein at nucleotide positions 275 and 360 respectively. The cloning vector does not contain these recognition sequences. Consequently, only two fragments were seen following restriction enzyme digestion with *PmlI* and *Bsu36*I, one corresponding to the vector fragment, the other corresponding to the ∼85 base pair fragment liberated from within the protein coding sequence. This sequence was replaced by any DNA sequence encoding the amino acid substitutions of the present invention. These DNA sequences were synthesized chemically as two oligonucleotides with complementary bases and ends that are compatible with the ends generated by digestion with *PmlI* and *Bsu36*I. The chemically synthesized oligonucleotides were mixed in equimolar amounts (1-10 picomoles/microliter), heated to 95°C and allowed to anneal by slowly decreasing the temperature to 20-25°C. The annealed oligonucleotides were used in a standard ligation reaction. Ligation products were transformed into *E. coli* DH10B cells (GIBCO BRL) and the transformed cells are plated on TY plates containing 10 µg/mL of tetracycline (Sigma, St. Louis, MO). Colonies which grow up overnight are grown in liquid TY medium with 10 µg/mL of tetracycline and are used for plasmid isolation and DNA sequence analysis. Plasmids with the correct sequence are kept.

### Example 3

### Expression plasmid pHS787 (Protein of SEQ ID NO:6)

The pHS692 vector, prepared in a manner analogous to Example 1, (40 µg) was digested with 20 units of *PmlI* (New England Biolab, Beverly, MA) in "New England Biolab buffer 1" at 37°C for two hours. The buffer salts were adjusted to the "New England Biolab buffer 3" and 20 units of *BstXI* (New England Biolab) were added to begin the digestion, which proceeded for 2 hours at 55°C. The digest was treated with 20 units of alkaline phosphatase (Boehringer Mannheim, Indianapolis, IN) at 37°C for 30 minutes. The resulting digest was purified on a 1% agarose gel and the 4170 bp fragment was isolated using the freeze-squeeze method.

The oligonucleotides 13824 (5'-TGA GGC TTC CAG GAC TCC CCC CAG ACT GTC CAA TGT CTC CAG GCC ACT GGC GTC TGG CAA GTG GCA ACT TTT AGA GAA GGC CAG CAC-3' (SEQ ID NO:14) and 13825 (5'-GTG CTG GCC TTC TCT AAA AGT TGC CAC TTG CCA GAC GCC AGT GGC CTG GAG ACA TTG GAC AGT CTG GGG GGA GTC CTG GAA GCC-3' (SEQ ID NO:15) were kinased in the presence of lx kinase buffer, 50 mM Tris-HCl pH 8.0, 10 mM MgCl₂, 0.5 mM ATP, 1 mM DTT, and 5 units T4 polynucleotide kinase (GIBCO BRL) at 37°C for 30 minutes.

The *PmlI/BstXI* linearized pHS692 vector and 13824 and 13825 linker were ligated in the presence of lx Kinase buffer, 0.5 mM ATP and 1 unit T4 DNA ligase (GIBCO BRL) at 16°C overnight. The ligation products are transformed into *E. coli* BL21(DE3) (NOVAGEN) and colonies growing on TY plates supplemented with 10 µg/mL of tetracycline are analyzed. Plasmid DNA is isolated, then subjected to DNA sequencing on a PE-Applied Biosystems 370 DNA sequencer. Plasmids containing the expected ∼400bp *NdeI* to *BamHI* fragment are kept and called pHS787.

### Example 4

### Expression plasmid (Protein of SEQ ID NO:3)

Plasmid pOJ722, was digested with *Pm1I* and *Bsu36*I. A synthetic DNA fragment of the sequence 5'-SEQ ID NO:16: 
(SEQ ID NO:16)
TGAGGCTTCCAGGACTCCCCCCAGACTGTCCAATGTCTCCAGGCCACTGGCGTCTGGCAA
GTGGCAACTTTTAGAGAAGGCCAGCAC
 annealed with the sequence 5'-SEQ ID NO:17: (SEQ ID NO:17) 
GTGCTGGCCTTCTCTAAAAGTTGCCACTTGCCAGACGCCAGTGGCCTGGAGACATTGGAC
AGTCTGGGGGGAGTCCTGGAAGCC
 was inserted between the *PmlI* and the *Bsu36*I sites. Following ligation, transformation and plasmid isolation, the sequence of the synthetic fragment was verified by DNA sequence analysis.

The techniques of transforming cells with the aforementioned vectors are well-known in the art [Maniatis, T., *et al. Molecular Cloning: A Laboratory Manual,* 2^{nd} Ed., Cold Spring Harbor Press, Cold Spring Harbor Laboratory, Cold Spring Harbor, New York (1988); *Id., Current Protocols in Molecular Biology* (1989), and supplements thereof]. The techniques involved in the transformation of *E. coli* cells used in the preferred practice of the invention as exemplified herein are well known in the art. The precise conditions under which the transformed *E. coli* cells are cultured is dependent on the nature of the *E. coli* host cell line and the expression or cloning vectors employed. For example, vectors which incorporate thermoinducible promoter-operator regions, such as the c1857 thermoinducible lambdaphage promoter-operator region, require a temperature shift from about 30°C to about 40°C. in the culture conditions so as to induce protein synthesis.

In the preferred embodiment of the invention, *E. coli* K12 RV308 cells are employed as host cells, but numerous other cell lines are available, such as, but not limited to, *E. coli* K12 L201, L687, L693, L507, L640, L641, L695, L814 (*E. coli* B). The transformed host cells are then plated on appropriate media under the selective pressure of the antibiotic corresponding to the resistance gene present on the expression plasmid. The cultures are then incubated for a time and temperature appropriate to the host cell line employed.

Proteins that are expressed in high-level bacterial expression systems characteristically aggregate in granules or inclusion bodies which contain high levels of the overexpressed protein [Kreuger, J. K., *et al.,* in *Protein Folding,* Gierasch, L. M. and King, J., eds., American Association for the Advancement of Science Publication No. 89-18S, Washington, D.C., 136-142 (1990)]. Such protein aggregates must be dissolved to provide further purification and isolation of the desired protein product. [Kreuger, J. K., *et al., supra.*]. A variety of techniques using strongly denaturing solutions such as guanidinium-HCl and/or weakly denaturing solutions such as urea are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

Preferably, the present proteins are expressed with a leader sequence. One of ordinary skill in the art would recognize that numerous leader sequences are operable; however, the leader sequence is preferably Met-R₁- , wherein R₁ is any amino acid except Pro or is absent, so that the expressed proteins may be readily converted to the claimed protein with Cathepsin C, or other suitable aminopeptidases. Preferably, R₁ is Arg, Asp, or Tyr; and most preferably, the proteins are expressed with a Met-Arg leader sequence. Interestingly, the leader sequence does not significantly affect stability or activity of the protein. However, the leader sequence is preferably cleaved from the protein. Thus, the proteins of the Formula: Met-R₁-SEQ ID NO:1 are useful as biological agents and, preferably, as intermediates.

### Example 5

The protein of SEQ ID NO:3 with a Met-Arg leader sequence was expressed in *E. coli,* isolated and folded by techniques analogous to the previous Examples. The pH of the protein solution was reduced to pH 2.8. The Met-Arg leader sequence was cleaved by the addition of 6-10 milliunits dDAP per mg of protein (dDAP is the abbreviation for a dipeptidylaminopeptidase isolated from the slime mold, *Dicteostelium descoidium,* described by Atkinson, P. R., *et al.,* U.S. Patent No. 5,565,330, issued October 15, 1996). The conversion reaction was allowed to proceed for 2-8 hours at room temperature. The progress of the reaction was monitored by high performance reversed phase chromatography. The reaction was terminated by adjusting the pH to 8 with NaOH. The des(Met-Arg) protein was further purified by cation exchange chromatography in the presence of 7-8 M urea and size exclusion chromatography in PBS. Following final purification of the proteins by size exclusion chromatography the proteins were concentrated to 3-3.5 mg/mL in PBS.

The purification of the claimed proteins is by techniques known in the art and includes reversed phase chromatography, affinity chromatography, ion exchange and size exclusion chromatography.

The claimed proteins contain two cysteine residues. Thus, a disulfide bond may be formed to stabilize the protein. The present invention includes proteins of the Formula (I) wherein the Cys at position 96 is cross-linked to Cys at position 146 as well as those proteins without such disulfide bonds. Preferably, the Cys at position 96 is disulfide bonded to the Cys at position 146. In addition the proteins of the present invention may exist, particularly when formulated, as dimers, trimers, tetramers, and other multimers. Such multimers are included within the scope of the present invention.

The present invention provides a method for treating obesity. The method comprises administering to the organism an effective amount of anti-obesity protein in a dose between about 1 and 1000 µg/kg. A preferred dose is from about 10 to 100 µg/kg of active compound. A typical daily dose for an adult human is from about 0.5 to 100 mg. In practicing this method, compounds of the Formula (I) can be administered in a single daily dose or in multiple doses per day. The treatment regime may require administration over extended periods of time. The amount per administered dose or the total amount administered will be determined by the physician and depend on such factors as the nature and severity of the disease, the age and general health of the patient and the tolerance of the patient to the compound.

The instant invention further provides pharmaceutical formulations comprising compounds of the present invention. The proteins, preferably in the form of a pharmaceutically acceptable salt, can be formulated for parenteral administration for the therapeutic or prophylactic treatment of obesity. For example, compounds of the Formula (I) can be admixed with conventional pharmaceutical carriers and excipients. The compositions comprising claimed proteins contain from about 0.1 to 95% by weight of the active protein, preferably in a soluble form, and more generally from about 10 to 30%. Furthermore, the present proteins may be administered alone or in combination with other anti-obesity agents or agents useful in treating diabetes. For intravenous (i.v.) use, the protein is administered in commonly-used intravenous fluid(s) and administered by infusion. Such fluids, for example, physiological saline, Ringer's solution or 5% dextrose solution can be used. For intramuscular preparations, a sterile formulation, preferably a suitable soluble salt form of a protein of the Formula (I), for example the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate. Pharmaceutically acceptable preservatives such as an alkylparaben, particularly methylparaben, ethylparaben, propylparaben, or butylparaben or chlorobutanol are preferably added to the formulation to allow multi-dose use. Significantly, the claimed proteins are also stable in the presence of a phenolic preservative, such as, m-cresol or phenol. The stability of the proteins in the presence of a phenolic preservative offers advantages in pharmaceutical delivery, including, enhanced preservative effectiveness. The formulation is preferably prepared in the absence of salt to minimize the ionic strength of the formulation.

The ability of the present compounds to treat obesity is demonstrated *in vivo*, as follows.

### Biological Testing

Parabiotic experiments suggest that a protein is released by peripheral adipose tissue and that the protein is able to control body weight gain in normal, as well as obese mice [Coleman, D. L., *Diabetologia* 14:141-148 (1978)]. Therefore, the most closely related biological test is to inject the test article by any of several routes of administration, e.g., intravenous (i.v.), subcutaneous (s.c.), intraperitoneal (i.p.), or by minipump or cannula, and then to monitor food and water consumption, body weight gain, plasma chemistry or hormones (glucose, insulin, ACTH, corticosterone, GH, T4) over various time periods.

Suitable test animals include normal mice (ICR, etc.) and obese mice (*ob/ob,* Avy/a, KK-Ay, tubby, fat). The *ob/ob* mouse model of obesity and diabetes is generally accepted in the art as being indicative of the obesity condition. Controls for non-specific effects for these injections are done using vehicle with or without the active agent of similar composition in the same animal monitoring the same parameters or the active agent itself in animals that are thought to lack the receptor (db/db mice, fa/fa or cp/cp rats). Proteins demonstrating activity in these models will demonstrate similar activity in other mammals, particularly humans.

Since the target tissue is expected to be the hypothalamus where food intake and lipogenic state are regulated, a similar model is to inject the test article directly into the brain, for example, by injection via lateral or third ventricles (i.c.v.) or directly into specific hypothalamic nuclei, such as, the arcuate, paraventricular, or perifornical nuclei. The same parameters as above could be measured, or the release of neurotransmitters that are known to regulate feeding or metabolism could be monitored (e.g. NPY, galanin, norepinephrine, dopamine, β-endorphin release).

The representative proteins outlined in Examples 6 and 7 were prepared in accordance with the teachings and examples provided herein. The designation Met -Arg - indicates that the protein was prepared and tested with a Met-Arg leader sequence attached. Amino acid sequences of the proteins of the examples were confirmed by mass spectroscopy and/or amino acid analysis. The proteins were folded with the Cys residues cross-linked by a disulfide bond when tested. The ability of the present proteins to treat obesity in a *ob/ob* mouse is presented in Tables 1-3. Similar studies are accomplished *in vitro* using isolated hypothalamic tissue in a perifusion or tissue bath system. In this situation, the release of neurotransmitters or electrophysiological changes is monitored.

### Example 6

### Testing of Ob Proteins of SEQ ID NO:2, 3, and 6 in ob/ob Male Mice

Male ob/ob mice [Harlan, Ltd., Blackthorn, England] were housed in groups of 5 animals each and provided with Purina 5008 chow and water *ad libitum.* The mice were maintained on a reverse lighting schedule (lights off at 9:00 A.M., and on at 9:00 P.M.). The mice were weighed daily at 8:30 A.M. Their food and water consumption were determined at the same time. Treatment, as indicated below, were made following the morning weighing, just prior to lights out. The mice were treated once daily for 4 days.

| Group | Treatment (n=5) |
|---|---|
| 1 | PBS 0.2 mL/day, S.C. |
| 2 | Protein 30 µg/0.2 mL/day, S.C. |
| 3 | Protein 300 µg/0.2 mL/day, S.C. |

The effects of these treatments on food consumption and cumulative body weight change are illustrated for representative proteins of the present invention in Tables 1-3.

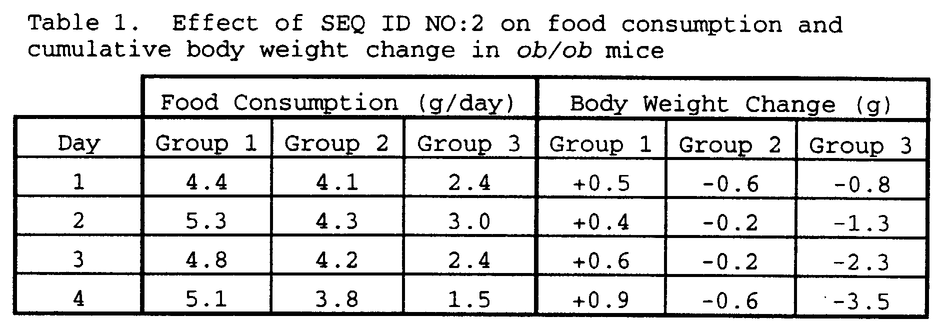

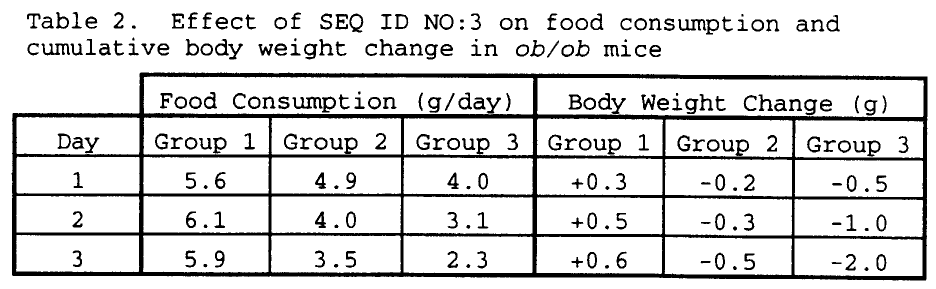

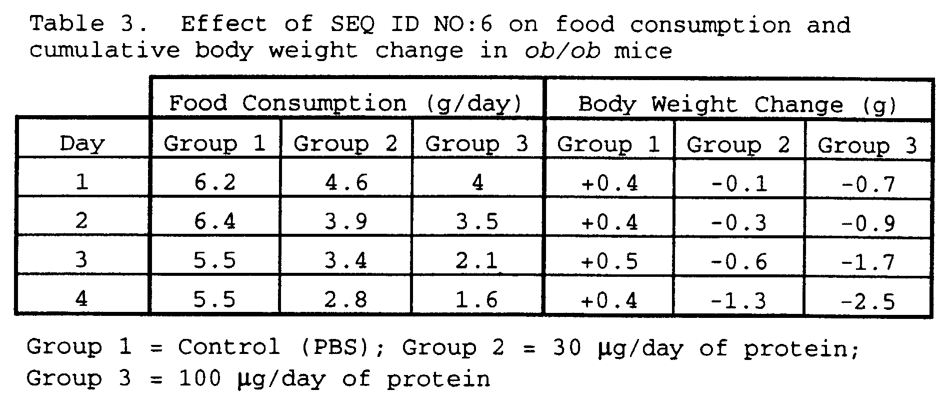

### Example 7

### Analysis of Aggregation by Dynamic Light Scattering (DLS)

The physical properties of the present compounds are demonstrated as follows. Based on availability of material, the solution analyzed by DLS was prepared in one of two ways. Material was supplied in solution from the last size exclusion chromatography purification step in PBS at approximately 1.5 mg/mL and was either concentrated to 3-5 mg/mL by diafiltration or dialyzed against water, lyophilized, and reconstituted to 3-5 mg/mL.

In the first method the protein solution at approximately 1.5 mg/mL was concentrated greater than 3-5 mg/mL in a small volume stir cell (10 mL) using an Amicon YM10, 25-mm membrane. This was conducted under cold room conditions (about 5°C). Protein concentration was determined by UV absorption and the solution diluted to 3.0 mg/mL with PBS (10-fold water dilution of 10x PBS without Ca/Mg, GIBCO BRL).

A second alternative method, used when lyophilization was feasible, consisted of dialyzing the solution of protein against water, using three to five exchanges of water at about 4°C. A typical dialysis membrane was the Spectra/Por-7 dialysis membrane, 2000 molecular weight cutoff membrane (Spectrum Medical Industries, Los Angeles, CA). The material was concentrated as above to 3 to 4 mg/mL and typically a 2 mg quantity was lyophilized in a 5 mL vial. For DLS analysis this plug was reconstituted with water to greater than 3.3 mg/mL or greater than 5 mg/mL. Several vials were typically pooled. Protein concentration was determined by UV at peak maximum (typically 280-nm). The protein concentration was diluted to about 3 or 5 mg/mL with a combination of water and 10x PBS (without Ca/Mg, GIBCO BRL) to yield a final PBS concentration of 1x.

The protein solution at 3.0 mg/mL or at 5 mg/mL in 1x PBS was adjusted to 7.4 with HCl/NaOH and passed through a 0.1 µm Anotop™-10 filter (Whatman International, Ltd., Maidstone, England) into a DLS cell. The average cumulant particle size was measured on a Brookhaven BI900 DLS instrument (Brookhaven Instruments, Holtsville, NY) with a Lexel argon-ion laser every 15 minutes using a 30 second duration at a 90° angle. A 488-nm filter with a 400 µm pinhole was used. At the 37°C incubation temperature a viscosity of 0.6915 centipoise (cP) and refractive index of 1.333 was used. The light-weighted average particle size was calculated by a cumulant method using the measured autocorrelation baseline.

The estimated time required for various anti-obesity protein compounds to reach an averaged light-weighted particle size of 50 nm in a PBS solution at pH 7.4 and 37°C is shown in Table 4. The averaged light-weighted particle size was determined from a cumulant analysis of a binodal distribution composed of monomeric and higher-order aggregate populations. The time necessary to achieve an average aggregate size of 50 nm was estimated by plotting size as a function of time. The proteins were folded with the Cys residues cross-linked by a disulfide bond when tested. For reference, the human Ob protein, and the protein of SEQ ID NO:18 are presented. The notation "u.d." signifies unable to determine and "n.d." signifies not determined. The protein of SEQ ID NO:18 has the sequence:

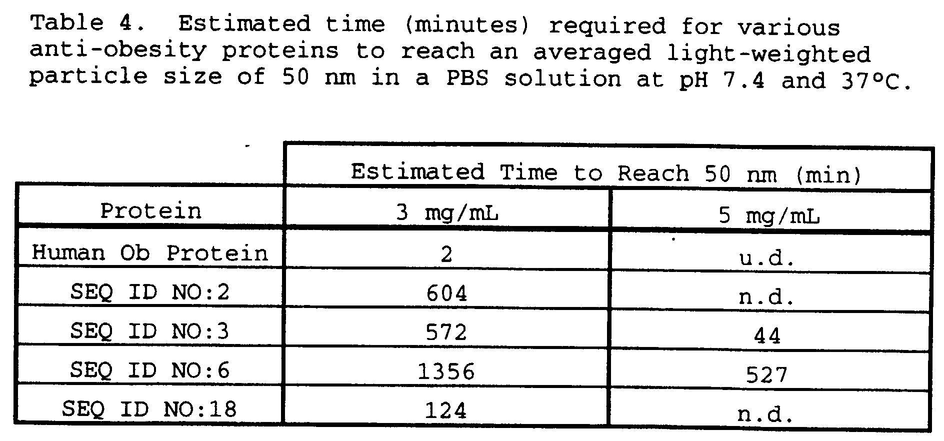

                    5                   10                  15
Met Arg Val Pro Ile Gln Lys Val Gln Asp Asp Thr Lys Thr Leu Ile

            20                  25                  30
Lys Thr Ile Val Thr Arg Ile Asn Asp Ile Ser His Thr Gln Ser Val

        35                  40                  45
Ser Ser Lys Gln Lys Val Thr Gly Leu Asp Phe Ile Pro Gly Leu His

    50                  55                  60
Pro Ile  Leu Thr Leu Ser Lys Met Asp Gln Thr Leu Ala Val Tyr Gln

65                  70                  75                  80
Gln Ile Leu Thr Ser Met Pro Ser Arg Asp Val Ile Gln Ile Ser Asn

                85                  90                  95
Asp Leu Glu Asn Leu Arg Asp Leu Leu His Val Leu Ala Phe Ser Lys

            100                 105                 110
Ser Cys His Leu Pro Trp Ala Ser Gly Leu Glu Thr Leu Asp Ser Leu

        115                 120                 125
Gly Gly Val Leu Glu Ala Ser Gly Tyr Ser Thr Glu Val Val Ala Leu

    130                 135                 140
Ser Arg Leu Gln Gly Ser Leu Gln Asp Met Leu Trp Gln Leu Asp Leu

145
Ser Pro Gly Cys  (SEQ ID NO:18)

The compounds are active in at least one of the above biological tests and are anti-obesity agents. As such, they are useful in treating obesity and those disorders implicated by obesity. However, the proteins are not only useful as therapeutic agents; one skilled in the art recognizes that the proteins are useful in the production of antibodies for diagnostic use and, as proteins, are useful as feed additives for animals. Furthermore, the compounds are useful for controlling weight for cosmetic purposes in mammals. A cosmetic purpose seeks to control the weight of a mammal to improve bodily appearance. The mammal is not necessarily obese. Such cosmetic use forms part of the present invention.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: Anti-Obesity Proteins

(iii) NUMBER OF SEQUENCES: 18

(iv) CORRESPONDENCE ADDRESS:
(A) ADDRESSEE: A. M. Denholm
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(2) INFORMATION FOR SEQ ID NO:1:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Modified-site
(B) LOCATION: 22
(D) OTHER INFORMATION: /note= "Xaa at position 22 is Asn
or Ser;"

(ix) FEATURE:
(A) NAME/KEY: Modified-site
(B) LOCATION: 28
(D) OTHER INFORMATION: /note= "Xaa at position 28 is Gln
or absent;"

(ix) FEATURE:
(A) NAME/KEY: Modified-site
(B) LOCATION: 72
(D) OTHER INFORMATION: /note= "Xaa at position 72 is Asn,
Gln, Glu, or Asp;"

(ix) FEATURE:
(A) NAME/KEY: Modified-site
(B) LOCATION: 73

(D) OTHER INFORMATION: /note= "Xaa at position 73 is Val
or Met"

(xi) SEQUENCE DESCRIPTION: SEQ ID NO:1:

Val Pro Ile Gln Lys Val Gln Asp Asp Thr Lys Thr Leu Ile Lys Thr
1               5                   10                  15

Ile Val Thr Arg Ile Xaa Asp Ile Ser His Thr Xaa Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Xaa Xaa Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:2:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:2:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Asp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:3:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:3:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Glu Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:4:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Glu Gln Leu Asp Leu Ser Pro
    130                 135                 140

Gly Cys
145

(2) INFORMATION FOR SEQ ID NO:5:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:5:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Glu Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Asp Gln Leu Asp Leu Ser Pro
    130                 135                 140

Gly Cys
145

(2) INFORMATION FOR SEQ ID NO:6:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:6:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asp Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Asp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:7:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:7:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys

85                    90                    95

Ser Leu Pro Lys Thr Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:8:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:8:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Lys Gln Leu Asp Leu Ser Pro
    130                 135                 140

Gly Cys
145

(2) INFORMATION FOR SEQ ID NO:9:

(i) SEQUENCE CHARACTERISTICS:

(A) LENGTH: 148 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:9:

Met Arg Val Pro Ile Gln Lys Val Gln Asp Asp Thr Lys Thr Leu Ile
1               5                   10                  15

Lys Thr Ile Val Thr Arg Ile Asn Asp Ile Ser His Thr Gln Ser Val
            20                  25                  30

Ser Ser Lys Gln Lys Val Thr Gly Leu Asp Phe Ile Pro Gly Leu His
        35                  40                  45

Pro Ile Leu Thr Leu Ser Lys Met Asp Gln Thr Leu Ala Val Tyr Gln
    50                  55                  60

Gln Ile Leu Thr Ser Met Pro Ser Arg Asp Val Ile Gln Ile Ser Asn
65                  70                  75                  80

Asp Leu Glu Asn Leu Arg Asp Leu Leu His Val Leu Ala Phe Ser Lys
                85                  90                  95

Ser Cys His Leu Pro Glu Ala Ser Gly Leu Glu Thr Leu Asp Ser Leu
            100                 105                 110

Gly Gly Val Leu Glu Ala Ser Gly Tyr Ser Thr Glu Val Val Ala Leu
        115                 120                 125

Ser Arg Leu Gln Gly Ser Leu Gln Asp Met Leu Trp Gln Leu Asp Leu
    130                 135                 140

Ser Pro Gly Cys
145

(2) INFORMATION FOR SEQ ID NO:10:

(i) SEQUENCE CHARACTERISTICS:
(A) LENGTH: 148 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:10:

Met Arg Val Pro Ile Gln Lys Val Gln Asp Asp Thr Lys Thr Leu Ile
1               5                   10                  15

Lys Thr Ile Val Thr Arg Ile Asn Asp Ile Ser His Thr Gln Ser Val
            20                  25                  30

Ser Ser Lys Gln Lys Val Thr Gly Leu Asp Phe Ile Pro Gly Leu His
        35                  40                  45

Pro Ile Leu Thr Leu Ser Lys Met Asp Gln Thr Leu Ala Val Tyr Gln
    50                  55                  60

Gln Ile Leu Thr Ser Met Pro Ser Arg Asp Val Ile Gln Ile Ser Asn
65                  70                  75                  80

Asp Leu Glu Asn Leu Arg Asp Leu Leu His Val Leu Ala Phe Ser Lys
                85                  90                  95

Ser Cys His Leu Pro Asp Ala Ser Gly Leu Glu Thr Leu Asp Ser Leu
            100                 105                 110

Gly Gly Val Leu Glu Ala Ser Gly Tyr Ser Thr Glu Val Val Ala Leu
        115                 120                 125

Ser Arg Leu Gln Gly Ser Leu Gln Asp Met Leu Trp Gln Leu Asp Leu
    130                 135                 140

Ser Pro Gly Cys
145

(2) INFORMATION FOR SEQ ID NO:11:

(i) SEQUENCE CHARACTERISTICS:
(A) LENGTH: 148 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:11:

Met Arg Val Pro Ile Gln Lys Val Gln Asp Asp Thr Lys Thr Leu Ile
1               5                   10                  15

Lys Thr Ile Val Thr Arg Ile Asn Asp Ile Ser His Thr Gln Ser Val
            20                  25                  30

Ser Ser Lys Gln Lys Val Thr Gly Leu Asp Phe Ile Pro Gly Leu His
        35                  40                  45

Pro Ile Leu Thr Leu Ser Lys Met Asp Gln Thr Leu Ala Val Tyr Gln
    50                  55                  60

Gln Ile Leu Thr Ser Met Pro Ser Arg Asn Val Ile Gln Ile Ser Asn
65                  70                  75                  80

Asp Leu Glu Asn Leu Arg Asp Leu Leu His Val Leu Ala Phe Ser Lys
                85                  90                  95

Ser Cys His Leu Pro Glu Ala Ser Gly Leu Glu Thr Leu Asp Ser Leu
            100                 105                 110

Gly Gly Val Leu Glu Ala Ser Gly Tyr Ser Thr Glu Val Val Ala Leu
        115                 120                 125

Ser Arg Leu Gln Gly Ser Leu Gln Asp Met Leu Trp Gln Leu Asp Leu
    130                 135                 140

Ser Pro Gly Cys
145

(2) INFORMATION FOR SEQ ID NO:12:

    (i) SEQUENCE CHARACTERISTICS:
        (A) LENGTH: 148 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:12:

Met Arg Val Pro Ile Gln Lys Val Gln Asp Asp Thr Lys Thr Leu Ile
1               5                   10                  15

Lys Thr Ile Val Thr Arg Ile Asn Asp Ile Ser His Thr Gln Ser Val
            20                  25                  30

Ser Ser Lys Gln Lys Val Thr Gly Leu Asp Phe Ile Pro Gly Leu His
        35                  40                  45

Pro Ile Leu Thr Leu Ser Lys Met Asp Gln Thr Leu Ala Val Tyr Gln
    50                  55                  60

Gln Ile Leu Thr Ser Met Pro Ser Arg Asn Val Ile Gln Ile Ser Asn
65                  70                  75                  80

Asp Leu Glu Asn Leu Arg Asp Leu Leu His Val Leu Ala Phe Ser Lys
                85                  90                  95

Ser Cys His Leu Pro Asp Ala Ser Gly Leu Glu Thr Leu Asp Ser Leu
            100                 105                 110

Gly Gly Val Leu Glu Ala Ser Gly Tyr Ser Thr Glu Val Val Ala Leu
        115                 120                 125

Ser Arg Leu Gln Gly Ser Leu Gln Asp Met Leu Trp Gln Leu Asp Leu
    130                 135                 140

Ser Pro Gly Cys
145

(2) INFORMATION FOR SEQ ID NO:13:

(i) SEQUENCE CHARACTERISTICS:
(A) LENGTH: 458 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:13:

CATATGAGGG TACCTATCCA AAAAGTACAA GATGACACCA AAACACTGAT AAAGACAATA
60

GTCACAAGGA TAAATGATAT CTCACACACA CAGTCAGTCT CATCTAAACA GAAAGTCACA
120

GGCTTGGACT TCATACCTGG GCTGCACCCC ATACTGACAT TGTCTAAAAT GGACCAGACA
180

CTGGCAGTCT ATCAACAGAT CTTAACAAGT ATGCCTTCTA GAAACGTGAT ACAAATATCT
240

AACGACCTGG AGAACCTGCG GGATCTGCTG CACGTGCTGG CCTTCTCTAA AAGTTGCCAC
300

TTGCCATGGG CCAGTGGCCT GGAGACATTG GACAGTCTGG GGGGAGTCCT GGAAGCCTCA
360

GGCTATTCTA CAGAGGTGGT GGCCCTGAGC AGGCTGCAGG GGTCTCTGCA AGACATGCTG
420

TGGCAGCTGG ACCTGAGCCC CGGGTGCTAA TAGGATCC
458

(2) INFORMATION FOR SEQ ID NO:14:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:14:

TGAGGCTTCC AGGACTCCCC CCAGACTGTC CAATGTCTCC AGGCCACTGG CGTCTGGCAA
60

GTGGCAACTT TTAGAGAAGG CCAGCAC
87

(2) INFORMATION FOR SEQ ID NO:15:

(i) SEQUENCE CHARACTERISTICS:
(A) LENGTH: 84 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:15:

GTGCTGGCCT TCTCTAAAAG TTGCCACTTG CCAGACGCCA GTGGCCTGGA GACATTGGAC
60

AGTCTGGGGG GAGTCCTGGA AGCC
84

(2) INFORMATION FOR SEQ ID NO:16:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:16:

TGAGGCTTCC AGGACTCCCC CCAGACTGTC CAATGTCTCC AGGCCACTGG CGTCTGGCAA
60

GTGGCAACTT TTAGAGAAGG CCAGCAC
87

(2) INFORMATION FOR SEQ ID NO:17:

(i) SEQUENCE CHARACTERISTICS:
(A) LENGTH: 84 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:17:

GTGCTGGCCT TCTCTAAAAG TTGCCACTTG CCAGACGCCA GTGGCCTGGA GACATTGGAC
60

AGTCTGGGGG GAGTCCTGGA AGCC
84

(2) INFORMATION FOR SEQ ID NO:18:

(i) SEQUENCE CHARACTERISTICS:
(A) LENGTH: 148 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:18:

Met Arg Val Pro Ile Gln Lys Val Gln Asp Asp Thr Lys Thr Leu Ile
1               5                   10                  15

Lys Thr Ile Val Thr Arg Ile Asn Asp Ile Ser His Thr Gln Ser Val
            20                  25                  30

Ser Ser Lys Gln Lys Val Thr Gly Leu Asp Phe Ile Pro Gly Leu His
        35                  40                  45

Pro Ile Leu Thr Leu Ser Lys Met Asp Gln Thr Leu Ala Val Tyr Gln
    50                  55                  60

Gln Ile Leu Thr Ser Met Pro Ser Arg Asp Val Ile Gln Ile Ser Asn
65                  70                  75                  80

Asp Leu Glu Asn Leu Arg Asp Leu Leu His Val Leu Ala Phe Ser Lys
                85                  90                  95

Ser Cys His Leu Pro Trp Ala Ser Gly Leu Glu Thr Leu Asp Ser Leu
            100                 105                 110

Gly Gly Val Leu Glu Ala Ser Gly Tyr Ser Thr Glu Val Val Ala Leu
        115                 120                 125

Ser Arg Leu Gln Gly Ser Leu Gln Asp Met Leu Trp Gln Leu Asp Leu
    130                 135                 140

Ser Pro Gly Cys
145

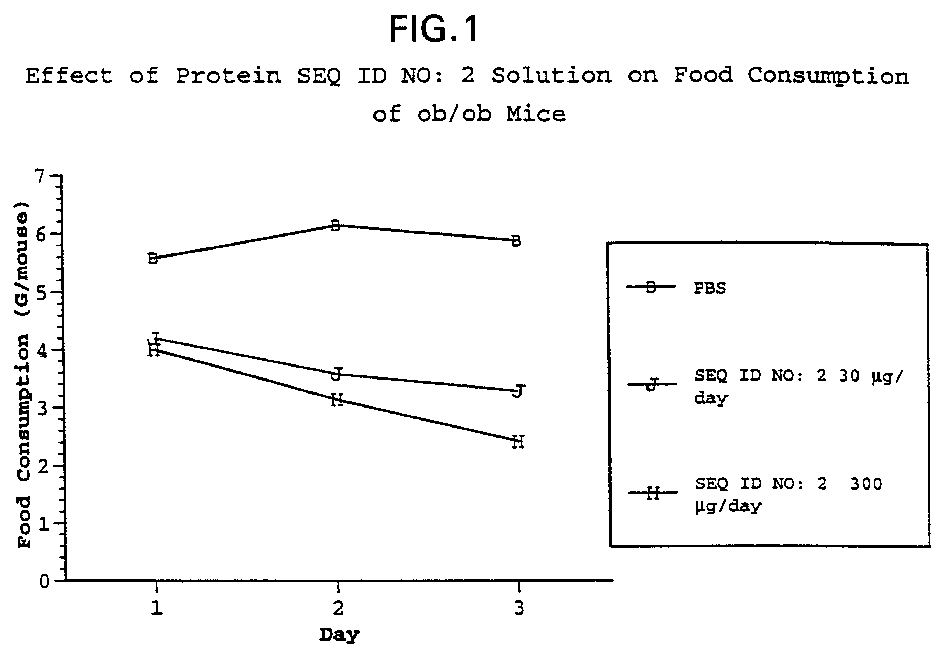

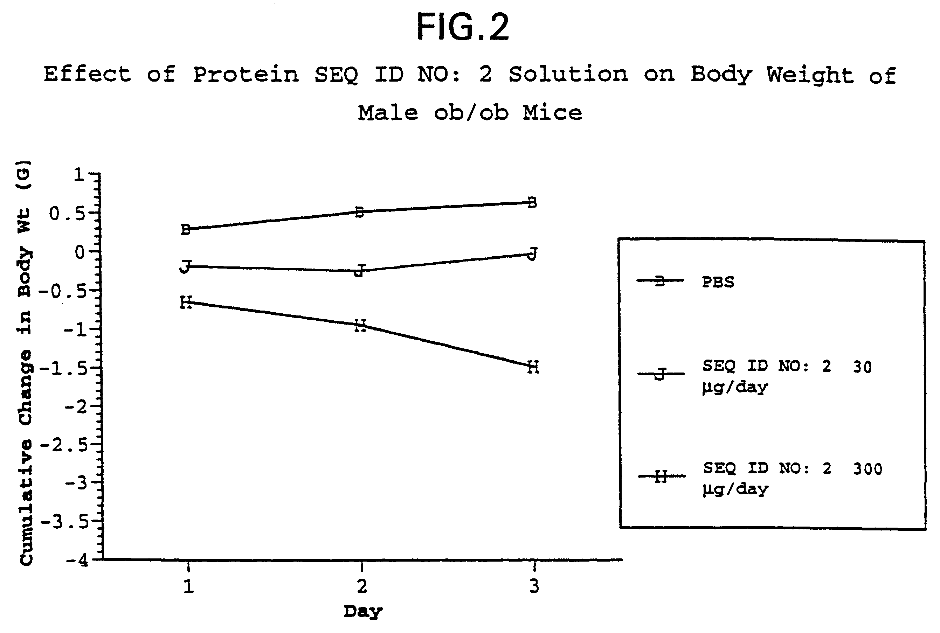

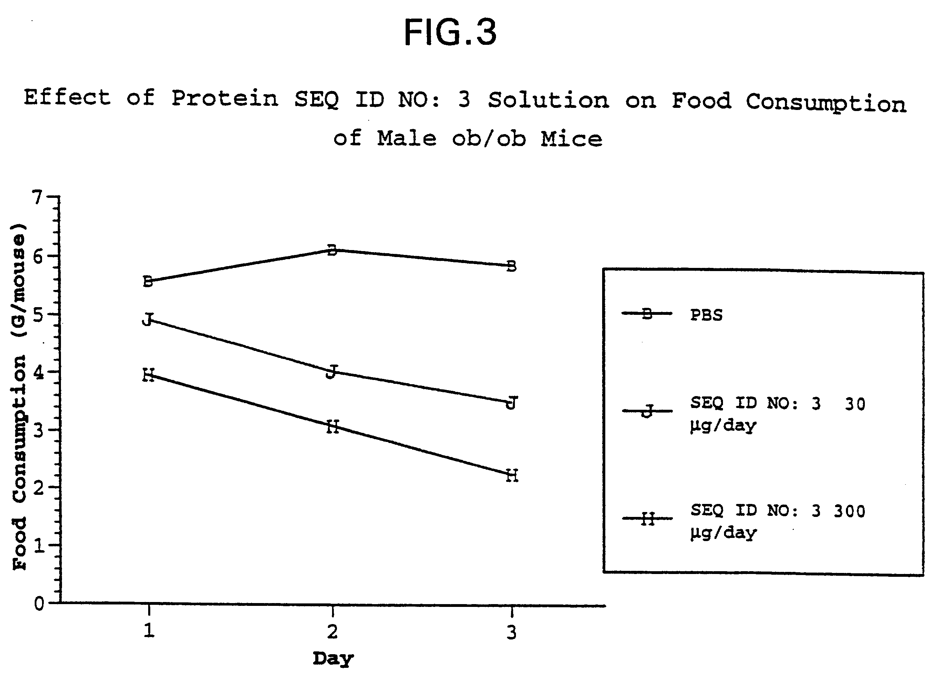

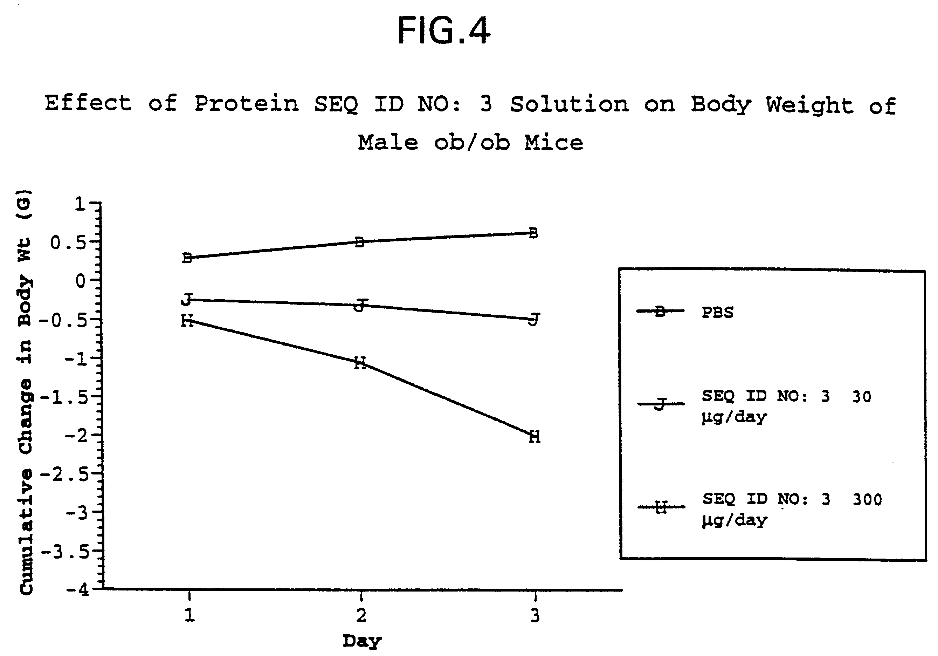

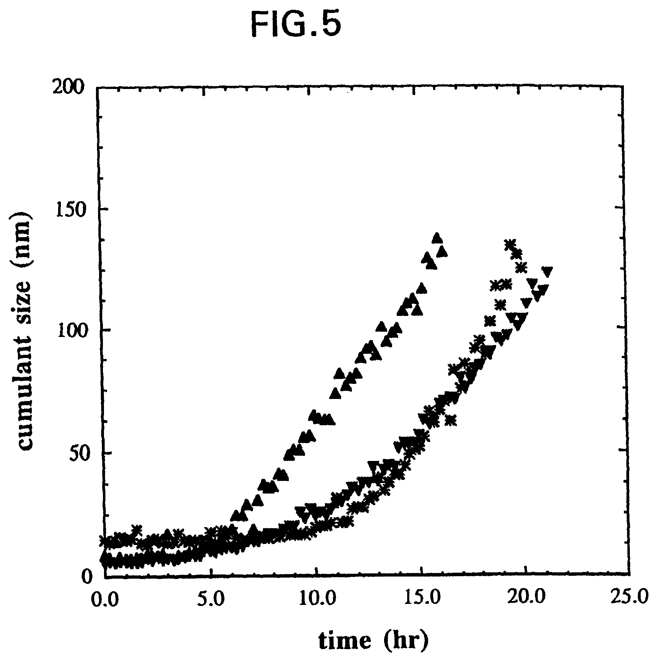

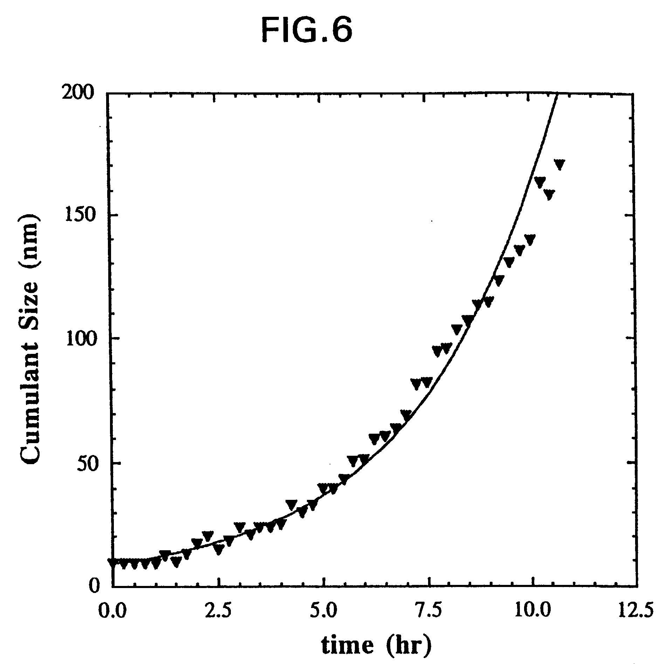

## Claims

1. A protein of the Formula (I): 
5                   10                  15
Val Pro Ile Gln Lys Val Gln Asp Asp Thr Lys Thr Leu Ile Lys Thr

            20                  25                  30
Ile Val Thr Arg Ile Xaa Asp Ile Ser His Thr Xaa Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Xaa Xaa Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys  (SEQ ID NO: 1)                                        (I)
 wherein:
Xaa at position 22 is Asn or Ser;
Xaa at position 28 is Gln or absent;
Xaa at position 72 in Asn, Gln, Glu, or Asp;
Xaa at position 73 is Val or Met;
said protein having at least one of the following replacements:
Trp at position 100 is replaced with Glu, Asp, His, Lys, or Arg; or
Trp at position 138 is replaced with Glu, Asp, His, Lys, or Arg;
or a pharmaceutically acceptable salt thereof.

2. The protein of Claim 1, wherein Cys at position 96 is disulfide bonded to Cys at position 146.

3. The protein of Claim 2, wherein
Xaa at position 22 is Asn;
Xaa at position 28 is Gln;
Xaa at position 72 in Asn or Asp; and
Xaa at position 73 is Val.

4. The protein of Claim 3, wherein Trp at position 100 is replaced with Glu or Asp.

5. The protein of Claim 2 which has a sequence selected from the group consisting of SEQ ID NO:2, SEQ ID NO:3, SEQ ID NO:4, SEQ ID NO:5, SEQ ID NO:6, SEQ ID NO:7, and SEQ ID NO:8.

6. A protein consisting of the protein as claimed in any one of Claims 1 to 5, and a leader sequence, wherein, the leader sequence is bonded to the N-terminal of the protein, or a pharmaceutically acceptable salt thereof.

7. The protein of Claim 6, wherein the leader sequence is Met-R₁, wherein R₁ is absent or is any amino acid except Pro.

8. The protein of Claim 7, wherein the leader sequence is Met-Arg, Met-Asp, or Met-Tyr.

9. The protein of Claim 8 which has a sequence selected from the group consisting of SEQ ID NO:9, SEQ ID NO:10, SEQ ID NO:11, and SEQ ID NO:12.

10. A pharmaceutical formulation, comprising as an active agent, a protein as claimed in any one of Claims 1 to 9, or a pharmaceutically acceptable salt thereof, together with one or more pharmaceutically acceptable diluents, carriers or excipients therefor.

11. A DNA polynucleotide compound comprising DNA that encodes a protein as claimed in any one of Claims 1 to 9.

12. A process for preparing a protein as claimed in any one of Claims 1 to 9, comprising:
(a) transforming a host cell with DNA that encodes the protein;
(b) culturing the transformed host cell such that the protein is expressed;
(c) optionally, enzymatically cleaving the leader sequence of the expressed protein; and
(d) recovering the protein.

13. A protein, as claimed in any one of Claims 1 to 9, or a pharmaceutically acceptable salt thereof, for use in manufacturing a medicament for treating obesity or conditions associated with obesity.

14. A protein as claimed in any one of Claims 1 to 9, or a pharmaceutically acceptable salt thereof, for use in treating obesity or conditions associated with obesity.
